# EUROPEAN PATENT APPLICATION

(11) **EP 4 159 713 A1**
(43) Date of publication of application: **05.04.2023**
(21) Application number: 21814338.6
(22) Date of filing: 24.05.2021
(51) Int. Cl.: C07C 253/00, C07C 255/53

(54) **PREPARATION METHOD FOR (4-ISOPROPOXY-2-METHYL) PHENYL ISOPROPYL KETONE**

(30) Priority: 28.05.2020 CN 202010468817
(71) Applicant: Adama Makhteshim Ltd., 8410001 Beer-Sheva (IL)
(72) Inventor: WU, Shaoxiang, Beijing 100094 (CN); GAO, Rui, Beijing 100094 (CN); WANG, Rui, Beijing 100094 (CN); WANG, Wenjun, Beijing 100094 (CN); LIU, Wei, Beijing 100094 (CN)
(74) Representative: Modiano, Gabriella Diana
(86) International application number: PCT/CN2021/095446
(87) International publication number: WO 2021/238839

(57) **Abstract**

The invention relates to a preparation method of (4-isopropoxy-2-methyl)phenyl isopropyl ketone, particularly the preparation method includes: reacting m-cresol with thiocyanate in the presence of a catalyst to obtain a product A; reacting the product A with haloisopropane in the presence of a base and a catalyst to obtain a product B; reacting the product B with isopropyl magnesium halide and treating to obtain (4-isopropoxy-2-methyl)phenyl isopropyl ketone. The purity of (4-isopropoxy-2-methyl)phenyl isopropyl ketone prepared through the above steps is more than 99%, and the total yield is more than 79%. The method according to the present invention avoids the use of toxic reagents and the generation of a large amount of acidic wastewater, reduces the reaction temperature, and improves the reaction yield. The process route is simple and efficient, and the cost is reduced. The purity of the resulting product is high, the production security is greatly improved, and the method is easy to industrialize.

## Description

### TECHNICAL FIELD

The invention relates to the field of organic chemistry, particularly relates to a preparation method of (4-isopropoxy-2-methyl)phenyl isopropyl ketone.

### BACKGROUND TECHNIQUE

The target compound (4-isopropoxy-2-methyl)phenyl isopropyl ketone) (Formula IV) is an intermediate of a fungicide isofetamid. Isofetamid is the latest broad-spectrum fungicide of succinate dehydrogenase inhibitors (SDHI) developed by Ishihara, it has both the protective, systemic action and the therapeutic effect, and may adversely affect the respiration of plants and fungi. Isofetamid may be used to control foliage and soil borne diseases, preventing various diseases caused by *Botrytis cinerea* and *Sclerotinia sclerotiorum* on crops such as grapes, lettuce, rape, dwarf berries and turf.

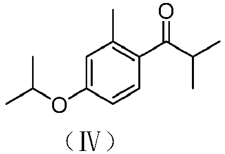

WO2006016708 discloses a chemical synthesis method of intermediate (4-hydroxy-2-methyl)phenyl isopropyl ketone of formula (V), and the reaction process is as follows:

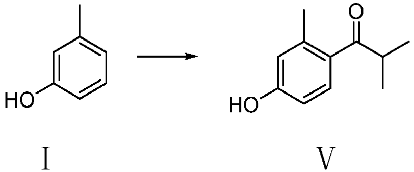

The method uses the m-cresol of formula (I) and isobutyryl chloride as raw materials, aluminum trichloride as a catalyst, and carbon disulfide as a solvent, after mixing and reacting, an intermediate of formula (V) is obtained through post-treatment. The intermediate of formula (V) may react with haloisopropane to obtain a product of formula (IV). In the process of synthesizing the intermediate of formula (V), the selectivity of the reaction is very poor, the yield is low; and the reaction will produce a large amount of acidic wastewater, which is not environmentally friendly. The pure product of formula (V) is obtained by column chromatography, which is not conducive to industrial production.

In view of problems in the prior art that, the synthesis of formula (V) through Friedel-Crafts acylation reaction and the subsequent synthesis of formula (IV) through etherification have low yield, large amount of the three wastes, and high cost. It would be very desirable to develop a new synthesis method suitable for industrial applications, which is simple, low-cost, high-yield, and environmentally friendly, so as to overcome the shortcomings of the existing technology.

### CONTENTS OF THE INVENTION

In order to solve the above-mentioned problems in the prior art, the present invention provides a simple, effective, easy-to-operate, and environment-friendly preparation method of (4-isopropoxy-2-methyl)phenyl isopropyl ketone. The purity of (4-isopropoxy-2-methyl)phenyl isopropyl ketone prepared by the method according to the present application is more than 99%, and the yield based on m-cresol is more than 79%.

The technical solutions adopted by the present invention are as follows:
A preparation method of (4-isopropoxy-2-methyl)phenyl isopropyl ketone, comprising the following steps:
(1) reacting m-cresol with thiocyanate in the presence of a catalyst, filtrating to obtain a filtrate and recovering the catalyst, and then concentrating and crystallizing the filtrate to obtain a product A;
(2) reacting the product A with haloisopropane in the presence of a base and a catalyst, filtrating to obtain a filtrate, and then concentrating the filtrate to obtain a product B;
(3) reacting the product B with isopropyl magnesium halide to obtain a reaction mixture, acidifying the reaction mixture and lefting to stand for layering to obtain an organic phase, sequentially heating and distilling the organic phase under reduced pressure to obtain (4-isopropoxy-2-methyl)phenyl isopropyl ketone.

Further, in step (1) the catalyst is any one or more selected from the group consisting of: fuming sulfuric acid, methanesulfonyl chloride, chlorosulfonic acid, and sulfonyl chloride.

Further, the catalyst is attached to a silica or aluminium trioxide carrier to form a solid catalyst that is recyclable.

Further, the catalyst is silica-supported chlorosulfonic acid or silica-supported sulfonyl chloride.

Further, in step (1) the thiocyanate is any one or more selected from the group consisting of: KSCN, NaSCN, and NH₄SCN.

Further, the thiocyanate is KSCN.

Further, in step (1) the reaction temperature is 50-120 °C, and the reaction time is 8-16 hours.

Further, in step (2) the base is any one or more selected from the group consisting of: potassium carbonate, sodium carbonate, potassium bicarbonate, sodium bicarbonate, potassium hydroxide, and sodium hydroxide.

Further, the base is potassium carbonate.

Further, in step (2) the catalyst is any one or more selected from the group consisting of: pyridine, 4-dimethylaminopyridine, DABCO (chemical name: 1,4-diazabicyclo[2.2.2]octane; aliase: triethylenediamine), Me-DABCO (chemical name: 2-methyl-1,4-Diazabicyclo[2.2.2]octane), and tetramethylammonium hydroxide.

Further, the catalyst is tetramethylammonium hydroxide.

Further, in step (2) the haloisopropane is any one or more selected from the group consisting of: chloroisopropane, bromoisopropane, and iodoisopropane.

Further, the haloisopropane is chloroisopropane.

Further, in step (2) the reaction temperature is 25-80 °C, and the reaction time is 1-10 hours.

Further, in step (3) the isopropyl magnesium halide is any one or more selected from the group consisting of: isopropyl magnesium chloride, isopropyl magnesium bromide, and isopropyl magnesium iodide.

Further, the isopropyl magnesium halide is isopropyl magnesium chloride.

Further, the temperature of reacting the product B with the isopropyl magnesium halide is 45-70 °C, and the reaction time is 1-5 hours.

Further, in step (3) the specific operation of the acidification is:
adding dropwise the reaction mixture at a temperature of 15-40°C to 5-36% hydrochloric acid and stirring for 30-90 min.

Further, in step (3) the heating temperature is 80-150 °C, the temperature of the vacuum distillation is 134-138 °C, and the pressure is 1-5 Torrs.

The following are structures of the compounds involved in the preparation method according to the present invention:

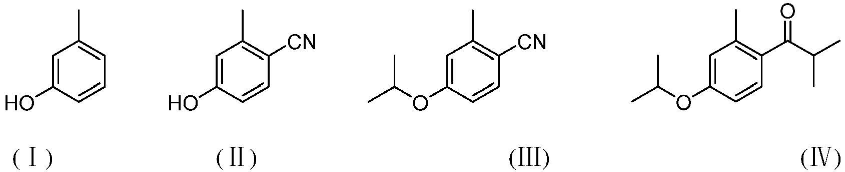

The beneficial effects of the present invention are as follows:
The preparation method of (4-isopropoxy-2-methyl)phenyl isopropyl ketone according to the present invention particularly includes: reacting m-cresol with thiocyanate in the presence of a catalyst, filtrating to obtain a filtrate and recovering the catalyst, and then concentrating and crystallizing the filtrate to obtain a product A; reacting the product A with haloisopropane in the presence of a base and a catalyst, filtrating to obtain a filtrate, and then concentrating the filtrate to obtain a product B; reacting the product B with isopropyl magnesium halide to obtain a reaction mixture, acidifying the reaction mixture and lefting to stand for layering to obtain an organic phase, desolvating the organic phase to obtain a concentrate, and distilling the concentrate under reduced pressure to obtain (4-isopropoxy-2-methyl)phenyl isopropyl ketone. The purity of (4-isopropoxy-2-methyl)phenyl isopropyl ketone prepared by the method according to the present application is more than 99%, and the yield based on m-cresol is more than 79%.

The invention uses m-cresol and thiocyanate to introduce a cyano group of the product A in the presence of a catalyst, which avoids the use of toxic reagents such as CuCN, KCN or NaCN, reducing the reaction temperature, and being simple to operate; and the catalyst may be recycled by washing with a solvent, which reduces the cost. The present invention utilizes the reaction of the product B with isopropyl magnesium halide (i.e. the introduction of isopropyl acyl group through the reaction of Grignard reagent and cyano group) instead of the conventional Friedel-Crafts acylation reaction; the entire reaction process effectively avoids a large amount of acidic wastewater, improves the reaction yield, and reduces a series of environmental impacts caused by post-processing. Compared with the existing Friedel-Crafts acylation route, the present invention has simple operation, high yield, and less waste water generation; and it is more suitable for industrial production.

### SPECIFIC EMBODIMENTS

In order to make the objectives, technical solutions and advantages of the present invention clearer, the technical solutions of the present invention will be described in detail below. Obviously, the described examples are only a part of the examples of the present invention, rather than all the examples. Based on the examples of the present invention, all the other implementation manners obtained by those of ordinary skill in the art without creative work shall fall within the protection scope of the present invention.

The structural formulas of the chemicals involved in the examples are as follows:

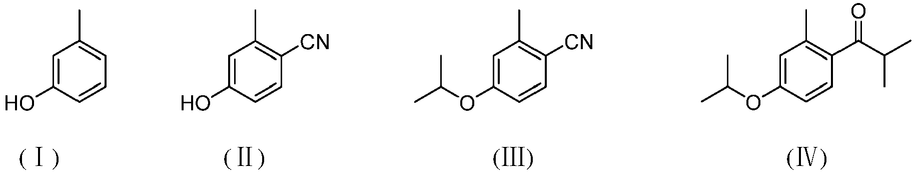

### Example 1

The example provides a preparation method of (4-isopropoxy-2-methyl)phenyl isopropyl ketone:
(1) 109g of m-cresol (formula I), 98g of potassium thiocyanate, and 314g of silica-supported chlorosulfonic acid are sequentially put into a flask equipped with a condenser, stirring and raising the temperature to 50 °C, then keeping warm and stirring to react for 16 hours. After finishing the reaction, 110g of toluene is added to the reaction mixture, lowering the temperature to 30 °C, filtering the mixture to obtain a filtrate, desolventizing the filtrate to obtain a concentrate and recover toluene; then 132g of n-butyl ether is added to the concentrate, raising the temperature until the concentrate is completely dissolved, and then lowering the temperature to 0 °C, filtering the mixture and drying the residue to obtain 113g of product A (formula II) with a purity of 99.0% and a yield of 84.0%.
(2) 300g of toluene, 63g of potassium carbonate, 100.8g of the product A, 0.15g of tetramethylammonium hydroxide are sequentially put into a flask equipped with a condenser, controlling the temperature at 50-55°C and adding dropwise 64.5g of chloroisopropane to the flask, after the dropwise addition, controlling the temperature at 25 °C and stirring to react for 10 hours. After finishing the reaction, the reaction mixture is filtered to obtain a filtrate, desolventizing the filtrate to obtain 133.8g of the concentrate of product B (formula III) with a content of 97.1% and a yield of 98.9% and recover toluene.
(3) 89.4g of the product B and 90g of tetrahydrofuran are sequentially put into a flask equipped with a condenser, controlling the temperature at 50-55 °C, and slowly adding 107g of tetrahydrofuran solution of isopropyl magnesium chloride, after the dropwise addition, controlling the temperature at 45 °C and stirring to react for 5 hours. After finishing the reaction, the temperature is controlled at 15 °C, and the reaction mixture is added dropwise to 20% hydrochloric acid, stirring for 90 minutes, and standing for layering to obtain an organic phase, then heating the organic phase to 80 °C to obtain a concentrate and recover tetrahydrofuran, and heating the concentrate to 140 °C and collecting the fraction at 134 °C at a pressure of 1 Torr to obtain 105.7 g of the product (formula IV) of the present invention, with a purity of 99.0% and a yield of 95.0%.

### Example 2

The example provides a preparation method of (4-isopropoxy-2-methyl)phenyl isopropyl ketone:
(1) 109g of m-cresol (formula I), 98g of potassium thiocyanate, and the catalyst recovered in Example 1 are sequentially put into a flask equipped with a condenser, stirring the mixture and raising the temperature to 120 °C, keeping warm and stirring to react for 8 hours. After finishing the reaction, 110g of toluene is added to the reaction mixture, lowering the temperature to 30 °C, filtering the mixture to obtain a filtrate, desolventizing the filtrate to obtain a concentrate and recover toluene; then 132g of n-butyl ether is added to the concentrate, raising the temperature until the concentrate is completely dissolved, and then lowering the temperature to 0 °C, filtering the mixture and drying the residue to obtain 122.4g of product A (formula II) with a purity of 99.0% and a yield of 83.6%.
(2) 300g of toluene, 80.3g of sodium carbonate, 100.8g of the product A, and 0.35g of 4-dimethylaminopyridine are sequentially put into a flask equipped with a condenser, controlling the temperature at 50-55°C and adding dropwise 64.5g of bromoisopropane to the flask, after the dropwise addition, controlling the temperature at 80 °C and stirring to react for 1 hour. After finishing the reaction, the reaction mixture is filtered to obtain a filtrate, desolventizing the filtrate to obtain 132.3g of the concentrate of product B (formula III) with a content of 96.8% and a yield of 97.5% and recover toluene.
(3) 89.4g of the product B and 90g of tetrahydrofuran are sequentially put into a flask equipped with a condenser, controlling the temperature at 50-55 °C, and slowly adding 181g of tetrahydrofuran solution of isopropyl magnesium bromide, after the dropwise addition, controlling the temperature at 70 °C and stirring to react for 1 hour. After finishing the reaction, the temperature is controlled at 40 °C, and the reaction mixture is added dropwise to 30% hydrochloric acid, stirring for 30 minutes, and standing for layering to obtain an organic phase, then heating the organic phase to 80 °C to obtain a concentrate and recover tetrahydrofuran, and heating the concentrate to 150 °C and collecting the fraction at 138 °C at a pressure of 5 Torrs to obtain 106.0 g of the product (formula IV) of the present invention, with a purity of 99.2% and a yield of 95.5%.

### Example 3

The example provides a preparation method of (4-isopropoxy-2-methyl)phenyl isopropyl ketone
(1) 109g of m-cresol (formula I), 98g of potassium thiocyanate, and 370g of silica-supported sulfuryl chloride are sequentially put into a flask equipped with a condenser, stirring and raising the temperature to 97.5 °C, then keeping warm and stirring to react for 12 hours. After finishing the reaction, 110g of toluene is added to the reaction mixture, lowering the temperature to 30 °C, filtering the mixture to obtain a filtrate, desolventizing the filtrate to obtain a concentrate and recover toluene; then 132g of n-butyl ether is added to the concentrate, raising the temperature until the concentrate is completely dissolved, and then lowering the temperature to 0 °C, filtering the mixture and drying the residue to obtain 113.2g of product A (formula II) with a purity of 99.0% and a yield of 84.2%.
(2) 300g of toluene, 63g of potassium bicarbonate, 100.8g of the product A, and 0.42g of DABCO are sequentially put into a flask equipped with a condenser, controlling the temperature at 50-55°C and adding dropwise 64.5g of 2-chloropropane to the flask, after the dropwise addition, controlling the temperature at 62 °C and stirring to react for 3 hours. After finishing the reaction, the reaction mixture is filtered to obtain a filtrate, desolventizing the filtrate to obtain 132.2g of the concentrate of product B (formula III) with a content of 94.5% and a yield of 95.0% and recover toluene.
(3) 89.4g of the product B and 90g of tetrahydrofuran are sequentially put into a flask equipped with a condenser, controlling the temperature at 50-55 °C, and slowly adding 181g of tetrahydrofuran solution of isopropyl magnesium bromide, after the dropwise addition, controlling the temperature at 58 °C and stirring to react for 3 hours. After finishing the reaction, the temperature is controlled at 27.5 °C, and the reaction mixture is added dropwise to 36% hydrochloric acid, stirring for 60 minutes, and standing for layering to obtain an organic phase, then heating the organic phase to 80 °C to obtain a concentrate and recover tetrahydrofuran, and heating the concentrate to 145 °C and collecting the fraction at 136 °C at a pressure of 3 Torrs to obtain 106.0 g of the product (formula IV) of the present invention, with a purity of 99.2% and a yield of 95.5%.

### Example 4

The example provides a preparation method of (4-isopropoxy-2-methyl)phenyl isopropyl ketone:
(1) 109g of m-cresol (formula I), 152.2g of ammonium thiocyanate, and 370g of a catalyst (silica-supported chlorosulfonic acid) are sequentially put into a flask equipped with a condenser, stirring and raising the temperature to 97.5 °C, then keeping warm and stirring to react for 12 hours. After finishing the reaction, 110g of toluene is added to the reaction mixture, lowering the temperature to 30 °C, filtering the mixture to obtain a filtrate, desolventizing the filtrate to obtain a concentrate and recover toluene; then 132g of n-butyl ether is added to the concentrate, raising the temperature until the concentrate is completely dissolved, and then lowering the temperature to 0 °C, filtering the mixture and drying the residue to obtain 110.8g of product A (formula II) with a purity of 98.0% and a yield of 81.5%.
(2) 300g of toluene, 63g of sodium hydroxide, 100.8g of the product A, and 0.42g of Me-DABCO are sequentially put into a flask equipped with a condenser, controlling the temperature at 50-55 °C and adding dropwise 64.5g of iodoisopropane to the flask, after the dropwise addition, controlling the temperature at 62 °C and stirring to react for 3 hours. After finishing the reaction, the reaction mixture is filtered to obtain a filtrate, desolventizing the filtrate to obtain 132.1g of the concentrate of product B (formula III) with a content of 94.5% and a yield of 95.0% and recover toluene.
(3) 89.4g (0.5mol)of the product B and 90g of tetrahydrofuran are sequentially put into a flask equipped with a condenser, controlling the temperature at 50-55 °C, and slowly adding 107g (0.52mol) of tetrahydrofuran solution of isopropyl magnesium chloride, after the dropwise addition, controlling the temperature at 55 °C and stirring to react for 4 hours. After finishing the reaction, the temperature is controlled at 25 °C, and the reaction mixture is added dropwise to 10% hydrochloric acid, stirring for 60 minutes, and standing for layering to obtain an organic phase, then heating the organic phase to 80 °C to obtain a concentrate and recover tetrahydrofuran, and heating the concentrate to 147 °C and collecting the fraction at 137 °C at a pressure of 4 Torrs to obtain 105.7 g of the product (formula IV) of the present invention, with a purity of 99.2% and a yield of 95.0%.

### Comparative Example

The target compound (4-isopropoxy-2-methyl)phenyl isopropyl ketone is synthesized by using existing technology, and the steps are as follows:
(1) 109g of m-cresol (formula I), 161.5g of anhydrous aluminum trichloride, and 800g of dichloromethane are sequentially put into a flask equipped with a condenser, controlling the temperature at 0-5 °C and slowly adding 117.6g of isobutyryl chloride to the flask, after the dropwise addition, controlling the temperature at 0-5 °C and stirring to react for 10 hours. After finishing the reaction, the reaction solution is slowly poured into 1000g of ice water, stirring for 0.5 hours, standing for layering to obtain an organic phase, desolventizing the organic phase to obtain a concentrate and recover dichloromethane, and then distilling the concentrate under reduced pressure to obtain 104.2g of bright yellow oily substance of intermediate (formula V), with a purity of 99.0% and a yield of 58.0%.
(2) 56.9g of the intermediate (formula V), 26.9g of potassium hydroxide, and 200g of ethanol are sequentially put into a flask equipped with a condenser, controlling the temperature at 60-65°C, and slowly adding 101g of 2-bromopropane dropwise, after the dropwise addition, controlling the temperature at 60-65 °C and stirring to react for 8 hours. After finishing the reaction, the reaction mixture is distilled to obtain a concentrate and recover ethanol, adding 100g of dichloromethane and 50g of water to the concentrate, stirring for 0.5 hours, then standing for layering to obtain an organic phase, desolventizing the organic phase to obtain a concentrate and recover dichloromethane, and distilling the concentrate under reduced pressure to obtain 60.5g of the target product (formula IV) with a purity of 99.0% and a yield of 86.0%. The total yield based on m-cresol is 50%.

Comparing the process of synthesizing the target product in Examples 1-4 and the Comparative Example, it can be concluded that during the process of synthesizing the intermediate in the Comparative Example, the selectivity of the reaction is very poor, the yield is low, and the reaction will produce a large amount of acidic wastewater, which is not environmentally friendly. Moreover, the intermediate is then etherified to synthesize the target product with low yield, large amount of the three wastes, and high cost; and the total yield based on m-cresol is only 50%. The purity of (4-isopropoxy-2-methyl)phenyl isopropyl ketone prepared by the technical solutions described in Examples 1-4 of the present application is more than 99%, and the total yield based on m-cresol is more than 79%. The present application uses m-cresol and thiocyanate to introduce a cyano group of the product A in the presence of a catalyst, which avoids the use of toxic reagents such as CuCN, KCN or NaCN, reducing the reaction temperature, and being simple to operate; and the catalyst may be recycled by washing with a solvent, which reduces the cost. The present invention utilizes the reaction of the product B with isopropyl magnesium halide (i.e. the introduction of isopropyl acyl group through the reaction of Grignard reagent and cyano group) instead of the conventional Friedel-Crafts acylation reaction; the entire reaction process effectively avoids a large amount of acidic wastewater, improves the reaction yield, and reduces a series of environmental impacts caused by post-processing. Compared with the existing Friedel-Crafts acylation route, the present invention has simple operation, high yield, and less waste water generation; and it is more suitable for industrial production.

The above are only specific embodiments of the present invention, but the protection scope of the present invention is not limited thereto. The modifications or substitutions that may be easily conceived by any person skilled in the art within the technical scope disclosed by the present invention should be covered within the protection scope of the present invention. Therefore, the protection scope of the present invention should be subject to the protection scope of the claims.

## Claims

1. A preparation method of (4-isopropoxy-2-methyl)phenyl isopropyl ketone, **characterized in that** it includes the following steps:
(1) reacting m-cresol with thiocyanate in the presence of a catalyst, filtrating to obtain a filtrate and recovering the catalyst, and then concentrating and crystallizing the filtrate to obtain a product A;
(2) reacting the product A with haloisopropane in the presence of a base and a catalyst, filtrating to obtain a filtrate, and then concentrating the filtrate to obtain a product B;
(3) reacting the product B with isopropyl magnesium halide to obtain a reaction mixture, acidifying the reaction mixture and lefting to stand for layering to obtain an organic phase, sequentially heating and distilling the organic phase under reduced pressure to obtain (4-isopropoxy-2-methyl)phenyl isopropyl ketone.

2. The preparation method of (4-isopropoxy-2-methyl)phenyl isopropyl ketone according to claim 1, **characterized in that** in step (1), the catalyst is any one or more selected from the group consisting of: fuming sulfuric acid, methanesulfonyl chloride, chlorosulfonic acid, and sulfonyl chloride.

3. The preparation method of (4-isopropoxy-2-methyl)phenyl isopropyl ketone according to claim 2, **characterized in that** the catalyst is attached to a silica or aluminium trioxide carrier to form a solid catalyst that is recyclable.

4. The preparation method of (4-isopropoxy-2-methyl)phenyl isopropyl ketone according to claim 1, **characterized in that** in step (1), the thiocyanate is any one or more selected from the group consisting of: KSCN, NaSCN, and NH₄SCN; the reaction temperature is 50-120 °C; and the reaction time is 8-16 hours.

5. The preparation method of (4-isopropoxy-2-methyl)phenyl isopropyl ketone according to claim 1, **characterized in that** in step (2), the base is any one or more selected from the group consisting of: potassium carbonate, sodium carbonate, potassium bicarbonate, sodium bicarbonate, potassium hydroxide, and sodium hydroxide.

6. The preparation method of (4-isopropoxy-2-methyl)phenyl isopropyl ketone according to claim 1, **characterized in that** in step (2), the catalyst is any one or more selected from the group consisting of: pyridine, 4-dimethylaminopyridine, DABCO, Me-DABCO, and tetramethylammonium hydroxide.

7. The preparation method of (4-isopropoxy-2-methyl)phenyl isopropyl ketone according to claim 1, **characterized in that** in step (2), the haloisopropane is any one or more selected from the group consisting of: chloroisopropane, bromoisopropane, and iodoisopropane; the reaction temperature is 25-80 °C; and the reaction time is 1-10 hours.

8. The preparation method of (4-isopropoxy-2-methyl)phenyl isopropyl ketone according to claim 1, **characterized in that** in step (3), the isopropyl magnesium halide is any one or more selected from the group consisting of: isopropyl magnesium chloride, isopropyl magnesium bromide, and isopropyl magnesium iodide; the temperature of reacting the product B with the isopropyl magnesium halide is 45-70 °C; and the reaction time is 1-5 hours.

9. The preparation method of (4-isopropoxy-2-methyl)phenyl isopropyl ketone according to claim 1, **characterized in that** in step (3), the specific operation of the acidification is:
adding dropwise the reaction mixture at a temperature of 15-40°C to 5-36% hydrochloric acid and stirring for 30-90 min.

10. The preparation method of (4-isopropoxy-2-methyl)phenyl isopropyl ketone according to claim 1, **characterized in that** in step (3), the heating temperature is 80-150 °C; the temperature of the vacuum distillation is 134-138 °C; and the pressure is 1-5 Torrs.
